# EUROPÄISCHE PATENTSCHRIFT

(11) **EP 0 553 445 B1**
(45) Veröffentlichungstag und Bekanntmachung des Hinweises auf die Patenterteilung: **24.03.1999**
(21) Anmeldenummer: 92120817.9
(22) Anmeldetag: 05.12.1992
(51) Int. Cl.: G01N 21/88, G01N 33/36, G01N 21/89, D01H 13/22, B65H 63/06

(54) **Vorrichtung zur Detektion von Verunreinigungen, insbesondere Fremdfasern in einem langgestreckten, textilen Gebilde**
Device for detecting impurities, especially foreign fibres in elongated textile articles
Dispositif de détection des impuretés, notamment des fibres étrangères dans des textiles allongés

(30) Priorität: 31.01.1992 CH 283/92
(43) Veröffentlichungstag der Anmeldung: 04.08.1993
(73) Patentinhaber: Gebrüder Loepfe AG, CH-8623 Wetzikon (CH)
(72) Erfinder: Scheinhütte, Hans-Jürgen, CH-8834 Schindellegi (CH)
(74) Vertreter: Blum, Rudolf Emil Ernst

(56) Entgegenhaltungen:
- EP-A- 0 197 763
- WO-A-89/05468
- WO-A-93/13407
- DE-A- 4 021 487

## Beschreibung

Die Erfindung betrifft eine Vorrichtung zur Detektion von Verunreinigungen, insbesondere Fremdfasern, in einem langgestreckten, textilen Gebilde, wie z.B. einem textilen Garn oder einem Faden.

Vor dem Verspinnen werden Rohfasern, wie z.B. Baumwollfasern, mittels Kardiermaschinen mechanisch gereinigt. Grobe Verunreinigungen, wie zum Beispiel Schalenrückstände der Baumwolle, können dadurch eliminiert werden. Trotz der perfektionierten, mechanischen Reinigung ist jedoch nicht auszuschliessen, dass kleine Verunreinigungen, wie z.B. Fremdfasern, im gesponnenen Faden auftreten.

Es ist schon länger bekannt, störende Garnfehler, d.h. Verdickungen oder Verdünnungen des Garns zu detektieren und zu beseitigen. Die sogenannten Garnreiniger schneiden das entsprechende Garnstück automatisch heraus.

In neuerer Zeit sind Bemühungen im Gang, nicht nur Garnfehler, sondern auch Verunreinigungen des Garns, insbesondere durch Fremdfasern, zu erkennen und zu beseitigen.

Eine solche Vorrichtung ist z.B. aus der Europäischen Patentschrift Nr. 0 197 763 bekannt. Ihr Messprinzip beruht darauf, den Faden durch opake Bereiche hindurch diffus so zu beleuchten, dass er sich nicht vom Hintergrund unterscheidet und dadurch für einen Lichtsensor unsichtbar wird, solange keine Verunreinigung auftritt. Damit kann insbesondere der Einfluss der Fadendicke auf das Sensorsignal ausgeschaltet werden.

Die in der genannten Patentschrift beschriebene Lösung besitzt den Nachteil, dass der Faden nicht in die Vorrichtung eingelegt werden kann, wie es sonst bei Garnreinigern üblich ist. Dies deshalb, weil der Faden auf dem Grund eines abgewinkelten Kanals verläuft, der den Zutritt von Fremdlicht ausschliesst.

Eine solche Vorrichtung kann deshalb nicht zusammen mit herkömmlichen Fadeneinlege-Einrichtungen verwendet werden.

Es stellt sich auf diesem Hintergrund die Aufgabe, eine Vorrichtung der eingangs genannten Art so auszugestalten, dass die Oeffnung zum Einlegen des Fadens derjenigen von üblichen Garnreinigern entspricht.

Die Lösung dieser Aufgabe ergibt sich aus den Merkmalen in den Patentansprüchen.

Anders als in der Vorrichtung gemäss EP- Nr. 0 197 763 wird das Licht nicht durch opake Bereiche in das Messfeld eingestrahlt, sondern gelangt aus den Lichtquellen durch Reflexionen an den verspiegelten und/oder diffus reflektierenden Aussenflächen des glasklaren Körpers zum Messfeld. Während bei der bekannten Anordnung lediglich das räumlich eng begrenzte Messfeld von diffusem Lichte erfüllt ist, stellt der an seiner Aussenfläche verspiegelte oder diffus reflektierende Körper aus transparentem Material einen wesentlich grösseren, von diffusem Licht erfüllten Raum dar. Entsprechend kann das Messfeld eine grössere Ausdehnung besitzen. Ferner ist der Lichtverlust durch die Einlegeöffnung relativ geringer. Die Lage des Fadens braucht damit nicht mehr eng geführt zu sein, was es erlaubt, ihn durch einen offenen, ebenen Schlitz im Körper laufen zu lassen, wie dies herkömmlichen Garnreinigern entspricht.

Im so gebildeten Raum, der von diffusem Licht erfüllt ist, reduziert jede lichtabsorbierende Verunreinigung auf dem Faden die Energiedichte, was sich in einem Lichtsensor, der im Messfeld angeordnet ist, als Signaländerung bemerkbar macht. Um das Sensorsignal von der Fadendicke im wesentlichen unabhängig zu machen, sind Lichtquellen beidseitig des Messfeldes derart angeordnet und aufeinander abgestimmt, dass die vom Sensor aufgenommene, reflektierte Helligkeit des Fadens beim Fehlen einer Verunreingiung im wesentlichen der Helligkeit des Messfeld-Hintergrundes entspricht.

Nachfolgend wird anhand der Figuren ein Ausführungsbeispiel der erfindungsgemässen Detektionsvorrichtung näher beschrieben. Dabei zeigt:
Figur 1 eine perspektivische Darstellung des Körpers aus optisch transparentem Material;
Figur 2 eine Aufsicht auf den Körper von Figur 1;
Figur 3 eine Schnittansicht entlang der Linie III - III in Figur 2, und
Figur 4 ein teilweise schematisches Schaltbild einer Ansteuerschaltung der Vorrichtung.

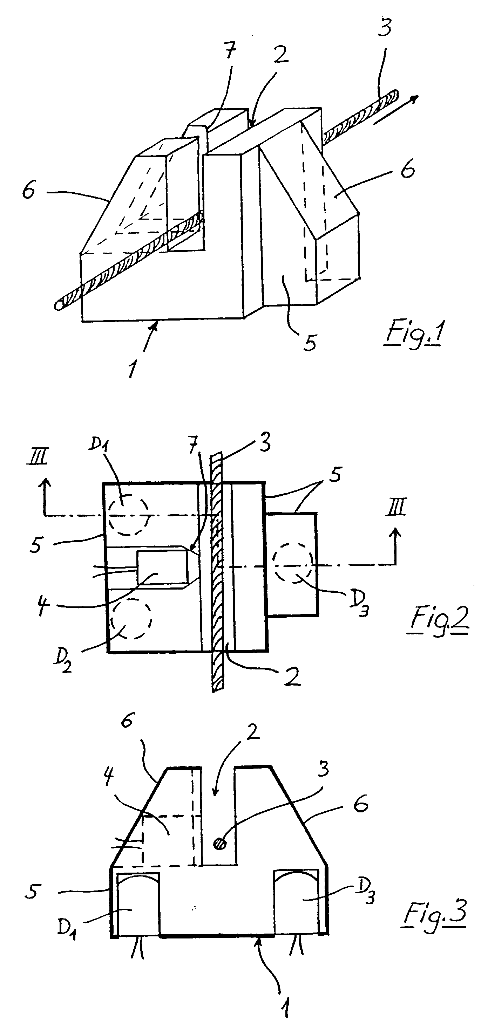

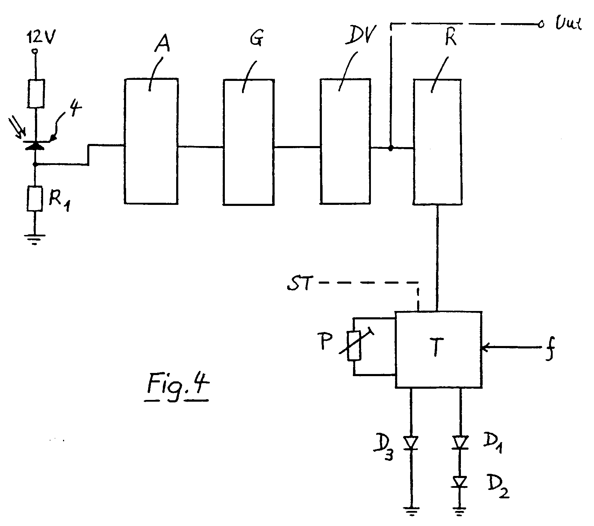

Wie sich insbesondere aus den Figuren 1 und 3 ergibt, besitzt die vergrössert dargestellte Detektionsvorrichtung einen Messkörper 1 aus glasklarem Kunststoff, der vorzugsweise als Kunststoff-Spritzgussteil ausgebildet ist und einen ebenen Schlitz 2 für den zu prüfenden Faden 3 besitzt. Der Schlitz 2, welcher das Messfeld definiert, bildet zugleich die Einlegeöffnung für den Faden, wie es bei normalen Garnreinigern üblich ist. Der Faden ist im Messfeld nicht fixiert, insbesondere kann er springen. Diesem Effekt wird durch eine Vergrösserung des Messfeldes begegnet. Wie sich aus den Figuren 2 und 3 ergibt, begrenzt der Lichtsensor 4 auf einer Seite das Messfeld, durch welches der zu prüfende Faden verläuft.

In den glasklaren Körper 1 wird mittels insgesamt drei lichtemittierenden Dioden D₁ bis D₃ Licht eingestrahlt. Anstelle der Dioden können andere Lichtquellen, z.B. Lichtleiter oder dergleichen vorgesehen sein. Das Licht wird an den Aussenflächen des Körpers 1, die gegen innen verspiegelt oder mit einer diffus reflektierenden Farbschicht 5 versehen sind, in den Körper hinein reflektiert und ergibt im Innern des Körpers, insbesondere im Messfeld, eine diffuse Beleuchtung.

Für die entsprechenden Reflexionen besitzt der Körper 1 an seiner den Dioden D₁ bis D₃ entgegengesetzten Seite dachartige Abschrägungen 6. Damit auch aus der Richtung des Sensors 4 Licht auf den Faden fällt, ist der Körper 1 beidseits des Sensors 4 so mit Lichtführungen 7 versehen, dass das Licht der beiden Dioden D₁ und D₂ schräg auf den Faden strahlt. Die dritte Diode D₃ ist auf der anderen Seite des Messfeldes 2 angeordnet und dient dazu, eine Hintergrundhelligkeit für den Sensor 4 zu erzeugen, die im wesentlichen der Helligkeit des vom Faden 3 in den Sensor 4 reflektierten Lichtes entspricht. Dadurch wird der Faden 3 bei entsprechendem Abgleich der Diode D₃, und wenn keine Verunreinigung vorhanden ist, für den Sensor 4 im wesentlichen "unsichtbar", so dass das Sensorsignal nicht durch die jeweilige Fadendicke beeinflusst wird.

Durch die Oberflächenverspiegelung ist der gesamte Körper 1 von Licht durchflutet. Dadurch erzeugt eine dunkle Faser im weissen Faden 3 auch dann ein Fehlersignal, wenn sie zum Detektor hin vom Faden verdeckt wird.

Ist der Abgleich korrekt durchgeführt worden, liefert ein weisser Faden beliebiger Dicke in Normalposition, d.h. in der Mitte vor dem Sensor 4, das gleiche Sensorsignal wie kein Faden. Die empfangene Lichtintensität ist also mit oder ohne Faden gleich.

In Figur 4 ist schematisch eine Schaltung zur Ansteuerung der beschriebenen Vorrichtung dargestellt. Das getaktete Licht aus den Dioden D₁ bis D₃ wird von der Fotodiode 4 empfangen und erzeugt am Widerstand R₁ eine Rechteckspannung. Diese wird von einem Verstärker A verstärkt. Seine Ausgangsspannung wird in einem Gleichrichter G gleichgerichtet. In einem weiteren Verstärker DV wird die durch das getaktete Licht verursachte Gleichspannung nochmals verstärkt.

Bei einer Herabsetzung der empfangenen Lichtintensität entsteht am Ausgang des Gleichspannungsverstärkers eine positive Spannung. Diese Spannung liegt auch am Eingang eines Reglers R für die Dioden D₁ bis D₃ und veranlasst diesen, seine Ausgangsspannung so lange zu ändern, bis die geregelte Beleuchtung am Ausgang des Gleichspannungsverstärkers wieder 0 V erzeugt.

Zur Steuerung der Dioden D₁ bis D₃ dient ein getakteter Treiber T, der vom Ausgang des Reglers R gespeist wird. Die Diode D₃, welche für den Abgleich der Hintergrundhelligkeit dient, kann, unabhängig vom Strom durch die Dioden, mit einem Potentiometer P und/ oder einer Steuerspannung ST abgeglichen werden.

Das Signal, das beim Auftreten von Verunreinigungen am Ausgang des Verstärkers DV auftritt, wird in bekannter Weise ausgewertet zur Erzeugung eines Fehlersignals beim Auftreten einer störenden Verunreinigung im geprüften Faden oder Garn. Die entsprechende Stelle wird dann mittels eines Garnreinigers herausgeschnitten. Danach kann der Faden dank des ebenen Messfeldschlitzes auf einfache Weise wieder in die beschriebene Detektionsvorrichtung eingelegt werden.

## Patentansprüche

1. Vorrichtung zur Detektion von Verunreinigungen in einem langgestreckten, textilen Gebilde, gekennzeichnet durch einen Messkörper (1) aus optisch transparentem Material, mit einem ebenen Schlitz (2), der ein Messfeld definiert, durch welches das textile Gebilde (3) in Längsrichtung hindurchführbar ist, und mit gegen innen verspiegelten und/oder diffus reflektierenden Aussenflächen (5), ferner gekennzeichnet durch eine oder mehrere Lichtquellen (D₁ bis D₃) zur Einstrahlung von Licht in den Messkörper, so dass das textile Gebilde im Messfeld (2) im wesentlichen allseitig von Licht beaufschlagt wird, sowie durch mindestens einen Lichtsensor (4), der auf einer Seite des Messfeldes (2) angeordnet ist, wobei die Lichtquellen (D₁ bis D₃) so plaziert und aufeinander abgestimmt sind, dass die vom Sensor (4) aufgenommene, reflektierte Helligkeit des textilen Gebildes beim Fehlen einer Verunreinigung im wesentlichen der Helligkeit des Messfeldhintergrundes entspricht.

2. Vorrichtung nach Anspruch 1, dadurch gekennzeichnet, dass der Messkörper (1) beidseitig des Messfeldschlitzes (2) Abschrägungen (6) aufweist zur Reflektion des Lichts aus den Lichtquellen (D₁ bis D₃) zum Messfeld hin.

3. Vorrichtung nach Anspruch 2, dadurch gekennzeichnet, dass die Lichtquellen (D₁ bis D₃) auf der den Abschrägungen (6) gegenüber liegenden Seite des Körpers (1) angeordnet sind.

4. Vorrichtung nach einem der vorangehenden Ansprüche, dadurch gekennzeichnet, dass zwei Lichtquellengruppen (D₁, D₂; D₃) vorgesehen sind, wobei die eine Lichtquellengruppe (D₁, D₂) im wesentlichen zur Erzeugung des Auflichts auf das textile Gebilde vorgesehen ist, dessen Reflektion durch den Lichtsensor (4) erfasst wird, und wobei die andere Gruppe (D₃) zum Abgleich der Hintergrundhelligkeit vorgesehen ist.

5. Vorrichtung nach Anspruch 4, dadurch gekennzeichnet, dass die beiden Lichtquellengruppen (D₁, D₂; D₃) jeweils auf entgegengesetzten Seiten des Messfeldschlitzes angeordnet sind.

6. Vorrichtung nach einem der Ansprüche 4 oder 5, dadurch gekennzeichnet, dass die Lichtquellengruppen (D₁, D₂; D₃) an eine Speiseschaltung (P, T) angeschlossen sind, mittels welcher das Helligkeitsverhältnis des durch die beiden Gruppen erzeugten Lichts einstellbar ist.

7. Vorrichtung nach einem der vorangehenden Ansprüche, dadurch gekennzeichnet, dass der Lichtsensor und die Lichtquellen (D₁ bis D₃) an eine Regelschaltung (R) angeschlossen sind, welche die Helligkeit der Lichtquellen so regelt, dass das Ausgangssignal der Vorrichtung bei Abwesenheit eines Fehlers auf einen bestimmten Spannungswert, vorzugsweise 0 Volt, eingestellt ist.

## Claims

1. Device for the detection of contaminations in an elongate, textile body characterised by a measuring body (1) of optically transparent material with a flat slit (2) defining a measuring field, through which the textile body (3) can be guided in longitudinal direction, and with inwardly mirror-like or diffusely reflecting outer surfaces (5), further characterised by one or more light sources (D₁ to D₃) for sending light into the measuring body such that in the measuring field (2) the textile body is hit by light from substantially all sides, as well as by at least one light sensor (4) arranged on one side of the measuring field (2), wherein the light sources are placed and adjusted to each other in such a way that the reflected brightness from the textile body recorded by the sensor (4) when no contamination is present substantially corresponds to the brightness of the background of the measuring field.

2. Device of claim 1, characterised in that the measuring body (1) comprises inclined surfaces (6) on both sides of the measuring field slit (2) for reflecting the light from the light sources (D₁ to D₃) towards the measuring field.

3. Device of claim 2, characterised in that the light sources (D₁ to D₃) are arranged on the side of the body (1) opposite to the inclined surfaces (6).

4. Device of one of the preceding claims characterised in that two groups of light sources (D₁, D₂; D₃) are provided, wherein one group of light sources (D₁, D₂) is provided substantially for generating light to be reflected from the textile body, the reflection of which is recorded by the light sensor (4), and wherein the other group (D₃) is provided for adjusting the background brightness.

5. Device of claim 4, characterised in that the two groups of light sources (D₁, D₂; D₃) are arranged on opposite sides of the measuring field slot.

6. Device of one of the claims 4 or 5 characterised in that the groups of light sources (D₁, D₂; D₃) are connected to a power supply (P, T), by means of which the ratio of brightness of the light generated by the two groups can be adjusted.

7. Device of one of the preceding claims characterised in that the light sensor and the light sources (D₁ to D₃) are connected to a regulating circuit (R), which regulates the brightness of the light sources such that the output signal of the device under absence of a defect is adjusted to a predefined voltage value, preferably 0 volts.

## Revendications

1. Dispositif pour la détection d'impuretés dans une structure textile allongée, caractérisé par une tête de mesure (1) en un matériau optiquement transparent, avec une fente plane (2) définissant un champ de mesure pouvant être traversé par la structure textile (3) se mouvant longitudinalement, et avec des surfaces extérieures (5) formant miroir et/ou réfléchissant de façon diffuse vers l'intérieur, caractérisé par une ou plusieurs sources lumineuses (D₁ à D₃) pour projeter de la lumière à l'intérieur de la tête de mesure de façon à essentiellement éclairer de tous les côtés la structure textile placée dans le champ de mesure (2), ainsi que par au moins un senseur de lumière (4) disposé sur un côté du champ de mesure (2), les sources lumineuses (D₁ à D₃) étant placées et mutuellement accordées de manière à ce qu'en l'absence d'impuretés la lumière réfléchie provenant de la structure textile et reçue par le senseur (4) correspond essentiellement à la luminosité de l'arrière-plan du champ de mesure.

2. Dispositif selon la revendication 1, caractérisé en ce que de chaque côté de la fente (2) formant le champ de mesure la tête de mesure (1) présente des chanfreins (6) pour réfléchir la lumière des sources lumineuses (D₁ à D₃) vers le champ de mesure.

3. Dispositif selon la revendication 2, caractérisé en ce que les sources de lumière (D₁ à D₃) sont disposées sur le côté de la tête (1) opposé aux chanfreins (6).

4. Dispositif selon une des revendications précédentes, caractérisé en ce qu'il est prévu deux groupes de sources lumineuses ((D₁, D₂; D₃) dont l'un (D₁, D₂) est destiné pour l'essentiel à l'éclairage direct de la structure textile, éclairage dont la partie réfléchie est détectée par le senseur de lumière (4), tandis que l'autre groupe (D₃) est prévu pour ajuster l'éclairage de l'arrière-plan.

5. Dispositif selon la revendication 4, caractérisé en ce que les deux groupes de sources lumineuses (D₁, D₂; D₃) sont placés de deux côtés opposés de la fente formant le champ de mesure.

6. Dispositif selon une des revendications 4 ou 5, caractérisé en ce que les groupes de sources lumineuses (D₁, D₂; D₃) sont connectés à une source d'alimentation (P, T) permettant de régler le rapport des intensités lumineuses produites par les deux groupes.

7. Dispositif selon une des revendications précédentes, caractérisé en ce que le senseur de lumière et les sources lumineuses (D₁ à D₃) sont connectés à un circuit de réglage (R) réglant l'intensité des sources lumineuses de manière à ce qu'en l'absence de défaut le signal de sortie du dispositif ait une tension déterminée, de préférence 0 Volt.
